# EUROPEAN PATENT APPLICATION

(11) **EP 4 696 301 A2**
(43) Date of publication of application: **18.02.2026**
(21) Application number: 25207758.1
(22) Date of filing: 14.09.2018
(51) Int. Cl.: A61K 9/00

(54) **A 19-NOR C3,3-DISUBSTITUTED C21-N-PYRAZOLYL STEROID AND METHODS OF USE THEREOF**

(30) Priority: 14.09.2017 US 201762558703 P; 31.01.2018 US 201862624680 P; 31.01.2018 US 201862624678 P; 05.04.2018 US 201862653189 P
(62) Divisional of application: 23150618.9
(71) Applicant: Sage Therapeutics, Inc., Cambridge, MA 02142 (US)
(72) Inventor: DOHERTY, James J., Bedford, 01730 (US); KANES, Stephen Jay, Swarthmore, 19081 (US); JONAS, Jeffrey M., Boston, 02116 (US); KAUL, Inderjit, Concord, 01742 (US)
(74) Representative: Coles, Andrea Birgit

(57) **Abstract**

Provided herein are methods for treating a sleep disorder, e.g., insomnia, in a subject, comprising administering to the subject an effective amount of a compound having the formula 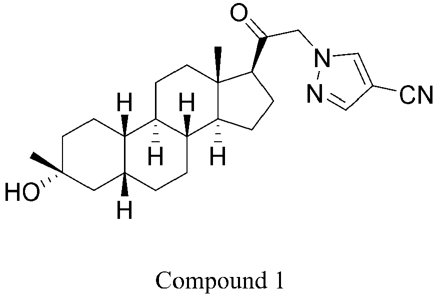 or a pharmaceutically acceptable salt thereof.

## Description

### Cross-Reference to Related Applications

This application claims priority to U.S.S.N. 62/588,703 filed September 14, 2017, U.S.S.N. 62/624,678 filed January 31, 2018, U.S.S.N. 62/624,680 filed January 31, 2018, and U.S. S.N. 62/653,189 filed April 5, 2018, the contents of which are incorporated herein by reference in their entirety.

### Field of the Invention

The present invention generally relates to methods of treating a sleep disorder such as insomnia by administering Compound 1 as described herein.

### Summary of the Invention

Described herein are methods of treating a sleep disorder in a subject, the method comprising administering to the subject a therapeutically effective amount of Compound 1 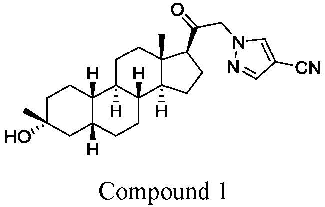 or a pharmaceutically acceptable salt thereof.

In one aspect, provided herein is a method of treating insomnia in a subject, the method comprising administering to the subject a therapeutically effective amount of Compound 1 or a pharmaceutically acceptable salt thereof. In some embodiments, the method comprises administering to the subject a therapeutically effective amount of Compound 1.

In some embodiments, Compound 1 is administered in the evening. In other embodiments, Compound 1 is administered prior to bedtime. In other aspects, Compound 1 is administered immediately before bedtime.

In some embodiments, methods of the invention increases non-REM sleep time. In some aspects, methods disclosed herein increase the time of stage 2 of sleep. In other aspects, methods disclosed herein increase the time of stage 3 of sleep. In further embodiments, methods disclosed herein increase the time of stage 2 and stage 3 of sleep. Increases in stages 2 and 3 can be measured in, for example, minutes. In some embodiments, methods disclosed herein increase the minutes the subject sleeps in stage 2 of sleep. In other embodiments, methods disclosed herein increase the minutes the subject sleeps in stage 3 of sleep. In further embodiments, methods disclosed herein increase the minutes the subject sleeps in stages 2 and 3.

In some embodiments, methods disclosed herein increase the time a subject sleeps in slow wave sleep. In some embodiments, methods disclosed herein do not significantly impact the time slept in REM sleep. In some aspects, methods disclosed herein increase the time slept in slow wave sleep, but does not significantly impact the time slept in REM sleep.

In some aspects, methods disclosed herein increase sleep efficiency. In some aspects, methods disclosed herein decreases wakefulness after sleep onset.

In some embodiments, methods disclosed herein increase total sleep time. In some further embodiments, methods disclosed herein decrease duration of awakenings.

In some embodiments, the therapeutically effective amount of Compound 1 is about 30 mg to about 45 mg. In some embodiments, Compound 1 is administered with food. In some aspects, Compound 1 is administered in one or more capsules. In some aspects, the therapeutically effective amount is administered across three capsules. In some embodiments, the effective amount of Compound 1 is administered once every 24-48 hours.

In some embodiments, methods disclosed herein do not significantly impact sleep latency. In some embodiments, methods disclosed herein do not cause a subject to fall asleep immediately. In some embodiments, in methods disclosed herein, sleep latency is not significantly different from placebo.

In some aspects, the subject is treated for insomnia regardless of having an underlying condition. In other embodiments, the subject does not have an underlying condition. In some aspects, the subject has an underlying condition. In some aspects, the insomnia is a side effect of another therapy. In some embodiments, the insomnia is a side effect of the subject's behavior. In some embodiments, the subject has a condition comorbid with insomnia.

In some aspects, provided herein are methods of increasing slow wave sleep in a subject comprising administering to a subject with a neurodegenerative disease or a central nervous system disorder a pharmaceutical composition comprising an effective amount of Compound 1 or a derivative thereof and a pharmaceutically acceptable carrier or excipient.

In some aspects, provided herein are methods of treating a sleep disorder in a subject comprising administering to a subject a pharmaceutical composition comprising an effective amount of Compound 1 or a derivative thereof and a pharmaceutically acceptable carrier or excipient.

In some aspects, provided herein are methods of increasing slow wave sleep (for example, increasing the minutes slept in this stage of sleep) in a subject comprising administering to a subject a pharmaceutical composition comprising an effective amount of Compound 1 or a derivative thereof and a pharmaceutically acceptable carrier or excipient.

In further embodiments, methods provided herein do not alter the number of sleep cycles a subject.

In some aspects, provided herein are methods of increasing the time slept in slow wave sleep in a subject without affecting the time of REM sleep comprising administering to a subject a pharmaceutical composition comprising an effective amount of Compound 1 or a derivative thereof and a pharmaceutically acceptable carrier or excipient.

### Brief Description of the Drawings

**FIG. 1** depicts a schematic of the study design of the trial as described in Example 1, optionally with Treatment Period 4.
**FIG. 2** depicts a schematic of the study design of the trial with Treatment Period 4 described in Example 1.

### Detailed Description of Certain Embodiments of the Invention

Described herein are methods of treating a sleep disorder, such as insomnia, in a subject, the method comprising administering to the subject a therapeutically effective amount of Compound 1 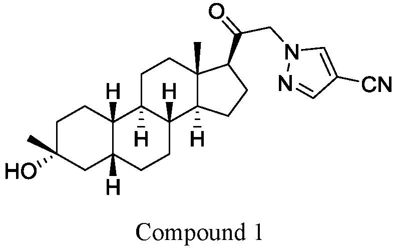 or a pharmaceutically acceptable salt thereof.

### Definitions

The term "AUC" refers to the area under the time/plasma concentration curve after administration of the pharmaceutical composition. AUC_{0-infinity} denotes the area under the plasma concentration versus time curve from time 0 to infinity; AUC₀₋ₜ denotes the area under the plasma concentration versus time curve from time 0 to time t. As used herein, AUC₀₋ₜ is the area under the plasma concentration versus time curve from the time of dosing to the last quantifiable concentration. It should be appreciated that AUC values can be determined by known methods in the art.

As used herein, the term "unit dosage form" is defined to refer to the form in which Compound 1 is administered to the subject. Specifically, the unit dosage form can be, for example, a pill, capsule, or tablet. Preferably, the unit dosage form is a capsule. The typical amount of Compound 1 in a unit dosage form useful in the invention is about 10 mg to about 100 mg, preferably about 20 mg to about 50 mg *(e.g.,* about 30 mg). In a preferred embodiment of the invention, the unit dosage form comprises about 30 mg of Compound 1 and is in the form of a capsule. In another preferred embodiment of the invention, the unit dosage form comprises about 45 mg of Compound 1 and is in the form of a capsule. Preferably, capsules which comprise about 30 mg or 45 mg of Compound 1, is administered to a subject once per day. In some embodiments, three capsules together comprise the 30 mg of Compound 1. In some embodiments, three capsules together comprises the 45 mg of Compound 1.

The term "Cₘₐₓ" refers to the maximum concentration of a therapeutic agent (*e.g.* Compound 1) in the blood (e.g. plasma) following administration of the pharmaceutical composition.

The term "tₘₐₓ" refers to the time in hours when Cₘₐₓ is achieved following administration of the pharmaceutical composition comprising the therapeutic agent *(e.g.* Compound 1).

As used herein, "solid dosage form" means a pharmaceutical dose(s) in solid form, e.g. tablets, capsules, granules, powders, sachets, reconstitutable powders, dry powder inhalers and chewables.

Where the use of the term "about" is before a quantitative value, the present teachings also include the specific quantitative value itself, unless specifically stated otherwise. As used herein, the term "about" refers to a ±10% variation from the nominal value unless otherwise indicated or inferred.

Definitions of specific functional groups and chemical terms are described in more detail below. The chemical elements are identified in accordance with the Periodic Table of the Elements, CAS version, *Handbook of Chemistry and Physics,* 75^{th} Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

As used herein, the term "modulation" refers to the inhibition or potentiation of GABA receptor function. A "modulator" (e.g., a modulator compound) may be, for example, an agonist, partial agonist, antagonist, or partial antagonist of the GABA receptor.

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

"Pharmaceutically acceptable salt" refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term "pharmaceutically acceptable cation" refers to an acceptable cationic counter-ion of an acidic functional group. Such cations are exemplified by sodium, potassium, calcium, magnesium, ammonium, tetraalkylammonium cations, and the like. See, *e.g.,* Berge, et al., J. Pharm. Sci. (1977) 66(1): 1-79.

A "subject" to which administration is contemplated includes, but is not limited to, humans *(i.e.,* a male or female of any age group, *e.g.,* a pediatric subject *(e.g,* infant, child, adolescent) or adult subject (e.g., young adult, middle-aged adult or senior adult)) and/or a non-human animal, *e.g.,* a mammal such as primates *(e.g.,* cynomolgus monkeys, rhesus monkeys), cattle, pigs, horses, sheep, goats, rodents, cats, and/or dogs. In certain embodiments, the subject is a human. In certain embodiments, the subject is a non-human animal. The terms "human," "patient," and "subject" are used interchangeably herein.

"Immediately before bedtime" means administering Compound 1 about 1 minute to about 30 minutes before a subject's bedtime, (e.g,, about 1 minute before bedtime, about 1-5 minutes before bedtime, about 5-10 minutes before bedtime, about 5-15 minutes before bedtime, about 10-20 minutes before bedtime, about 5-25 minutes before bedtime, or about 15-30 minutes before bedtime). In certain embodiments, Compound 1 is administered to the subject prior to bedtime. In some embodiments, Compound 1 is administered immediately before bedtime. In some embodiments, Compound 1is administered within about two hours before bedtime, preferably within about one hour before bedtime. In another embodiment, the Compound 1 is administered about two hours before bedtime. In a further embodiment, the Compound 1 is administered at least two hours before bedtime. In another embodiment, the Compound 1 is administered about one hour before bedtime. In a further embodiment, the Compound 1 is administered at least one hour before bedtime. In a still further embodiment, the Compound 1 is administered less than one hour before bedtime. In still another embodiment, the Compound 1 is administered immediately before bedtime.

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplates an action that occurs before a subject begins to suffer from the specified disease, disorder or condition ("prophylactic treatment").

As used herein, and unless otherwise specified, a "cycle of treatment" comprises administering a first dose of a neuroactive steroid, administering a second dose of the neuroactive steroid, and administering a third dose of the neuroactive steroid, said neuroactive steroid doses being sufficient to treat said subject.

In general, the "effective amount" of a compound refers to an amount sufficient to elicit the desired biological response, *e.g.,* to treat insomnia, to treat a CNS-related disorder, *e.g.,* a disorder as described herein *(e.g.,* tremor *(e.g.,* essential tremor); depression *(e.g.,* postpartum depression); or an anxiety disorder). As will be appreciated by those of ordinary skill in this art, the effective amount of a compound of the invention may vary depending on such factors as the desired biological endpoint, the pharmacokinetics of the compound, the disease being treated, the mode of administration, and the age, weight, health, and condition of the subject. An effective amount encompasses therapeutic and prophylactic treatment.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

### Pharmaceutical Compositions

In one aspect, the disclosure provides a pharmaceutical composition comprising a compound of the present invention (also referred to as the "active ingredient"), for example Compound 1, and a pharmaceutically acceptable excipient. In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

The pharmaceutical compositions provided herein can be administered by a variety of routes including, but not limited to, oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration. In preferred embodiments, Compound 1 is administering to a subject orally.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the onset of a CNS-disorder, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc,* or may be continued indefinitely, for example, for the rest of the subject's life. In certain embodiments, the chronic administration is intended to provide a constant level of the compound in the blood, *e.g*., within the therapeutic window over the extended period of time.

The pharmaceutical compositions of the present invention may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, *e.g*., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or preferably from about 1 to about 40% by weight) with the remainder being various vehicles or excipients and processing aids helpful for forming the desired dosing form.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

The compounds of the present invention can also be administered in sustained release forms or from sustained release drug delivery systems. A description of representative sustained release materials can be found in *Remington's Pharmaceutical Sciences.*

The present invention also relates to the pharmaceutically acceptable acid addition salt of a compound of the present invention. The acid which may be used to prepare the pharmaceutically acceptable salt is that which forms a non-toxic acid addition salt, *i.e.,* a salt containing pharmacologically acceptable anions such as the hydrochloride, hydroiodide, hydrobromide, nitrate, sulfate, bisulfate, phosphate, acetate, lactate, citrate, tartrate, succinate, maleate, fumarate, benzoate, para-toluenesulfonate, and the like.

### Methods of Use

Described herein are methods of treating a sleep disorder in a subject, the method comprising administering to the subject an effective amount of Compound 1or a pharmaceutically acceptable salt thereof.

Thus, in one aspect, provided herein is a method of treating insomnia in a subject, the method comprising administering to the subject a therapeutically effective amount of Compound 1 or a pharmaceutically acceptable salt thereof. In some embodiments, the method comprises administering to the subject a therapeutically effective amount of Compound 1. In some embodiments, the subject is between and including the ages of 18 and 64. In some embodiments, the compound is administered with food. In some embodiments, the therapeutically effective amount is about 30 mg. In some embodiments, the therapeutically effective amount is about 45 mg. In some embodiments, Compound 1 is administered in one or more capsules. In some embodiments, the therapeutically effective amount is administered across three capsules. In some embodiments, the subject is treated for insomnia regardless of any having underlying condition. In some embodiments, the subject does not have an underlying condition. In some embodiments, the subject has an underlying condition. In some embodiments, the insomnia is a side effect of some event. In some embodiments, the insomnia is a side effect of another therapy. In some embodiments, the insomnia is a side effect of the subject's behavior. In some embodiments, the subject has a condition comorbid with insomnia.

### Types of sleep

There are two basic types of sleep: rapid eye movement (REM) sleep and non-REM sleep (which has three different stages, Stage 1, Stage 2, and Stage 3). Each is linked to specific brain waves and neuronal activity. Sleep stages can be identified by monitoring a subject's brain electrical activity (e.g., brain waves). A person cycles through all stages of non-REM and REM sleep several times during a typical night, with increasingly longer, deeper REM periods occurring toward morning.

Stage 1 (N1) of non-REM sleep is the changeover from wakefulness to sleep. During this short period of relatively light sleep, heartbeat, breathing, and eye movements slow, and the muscles relax with occasional twitches. Also, brain waves begin to slow from their daytime wakefulness patterns.

Stage 2 (N2) of non-REM sleep is a period of light sleep before entering deeper sleep. The heartbeat and breathing slow further, and muscles relax further, too. The body temperature drops and eye movements stop. Brain wave activity slows but is marked by brief bursts of electrical activity.

Stage 3 (N3) of non-REM sleep is the period of deep sleep. It occurs in longer periods during the first half of the night. Heartbeat and breathing slow to their lowest levels during sleep. Muscles are relaxed and it may be difficult to awaken a person in this stage of sleep. Brain waves become even slower - this stage is also referred to as Slow Wave Sleep.

REM sleep first occurs about 90 minutes after falling asleep. Eyes move rapidly from side to side behind closed eyelids. Mixed frequency brain wave activity becomes closer to that seen in wakefulness. The breathing becomes faster and irregular, and heart rate and blood pressure increase to near waking levels. Most of the dreaming occurs during REM sleep, although some can also occur in non-REM sleep. The arm and leg muscles become temporarily paralyzed (thus preventing a person from acting out their dream). With aging, less time is spent in REM sleep.

One drawback of currently available insomnia drugs is that they disrupt REM sleep. This disruption leads to undesirable effects well known in the art.

In some embodiments, the compound of the present invention can be used to treat insomnia without disrupting REM sleep. In some embodiments, methods of the invention do not significantly impact the time of REM sleep. In some embodiments, methods of the invention, e.g. administering therapeutically effective amounts of Compound 1, are not significantly different from placebo in regards to the time of REM sleep.

In another embodiment, the compound of the present invention induces sleep in a patient in need thereof, wherein the sleep comprises a longer duration of non-REM sleep. In another embodiment, the compound of the present invention induces sleep in a patient in need thereof, wherein the sleep comprises a longer duration of non-REM sleep and a duration of REM sleep that is normal, i.e., of a duration comparable to that in a person of similar age and without insomnia.

In another embodiment, the compound of the present invention induces sleep in a patient in need thereof, wherein the sleep comprises a longer duration of slow wave sleep. In another embodiment, the compound of the present invention induces sleep in a patient in need thereof, wherein the sleep comprises a longer duration of slow wave sleep and a duration of REM sleep that is normal, i.e., of a duration comparable to that in a person of similar age and without insomnia.

### Examples

### Example 1. A Randomized, Double-Blind, Multiple Dose, 3-way Crossover, Exploratory Study to Assess the Pharmacodynamic Effects of Compound 1 Capsules in Healthy Adults Using a 5-hour Phase Advance Model of Insomnia.

### Number of Sites and Study Location: This study will take place at a single center in the US.

### Phase of Development: 1

### Objectives:

Primary:
   - To determine the overall effect of evening administration of Compound 1 on sleep
Secondary:
   - To identify changes in sleep parameters and architecture following evening administration of Compound 1
   - To assess the safety and tolerability of Compound 1 Capsules in healthy adults
   - To assess the pharmacokinetics (PK) of Compound 1 Capsules in healthy adults

### Exploratory:

- To assess the pharmacodynamic effect of Compound 1 Capsules on resting state electroencephalography (EEG) in healthy adults

### Endpoints:

Primary:
• Sleep efficiency, defined as the percentage of time in bed spent asleep, as determined by polysomnography (PSG).
Secondary:
*Efficacy*
   - Wakefulness after sleep onset (WASO) total and by quarter of the PSG recording,
   - Total sleep time (TST) and TST by quarter of the PSG recording,
   - Latency to persistent sleep (LPS),
   - Number of awakenings (NAW) and mean duration of awakenings, in total and by quarter of the PSG recording,
   - Subjective total sleep time (sTST),
   - Subjective wake after sleep onset (sWASO),
   - Subjective sleep latency (sSL),
   - Subjective sleep quality,
   - Minutes and percent of stage N1, N2, N3, and
   - Rapid eye movement (REM) sleep and latency to REM
Safety and Tolerability
   - Frequency, type, severity of adverse events; changes in vital sign assessments, clinical laboratory data, electrocardiogram (ECG) parameters, suicidality assessment via the Columbia Suicide Severity Rating Scale (C-SSRS), sleepiness assessment via Karolinska Sleepiness Scale (KSS), and psychomotor performance assessment via Digit Symbol Substitution Test (DSST)
Pharmacokinetic:
   - Area under the concentration-time curve from time zero to infinity (AUC0-∞)
   - Maximum plasma concentration (Cₘₐₓ)
   - Area under the concentration-time curve from time zero to last time point (AUC0-last)
   - Time to reach maximum concentration (tₘₐₓ)
Exploratory:
   - Absolute and relative changes in EEG alpha, theta, delta, beta, and gamma activity as determined by quantitative EEG analysis

### Methodology

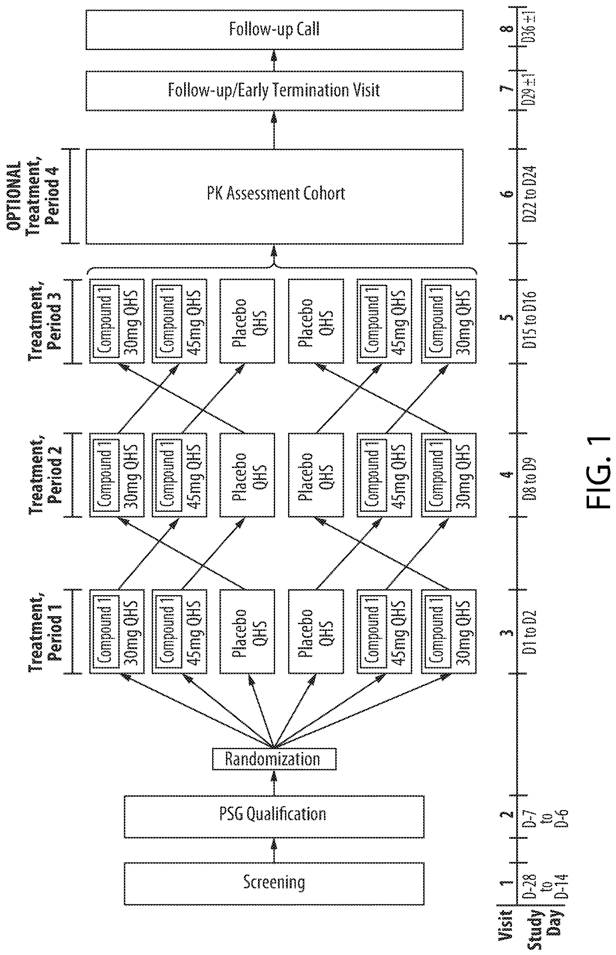

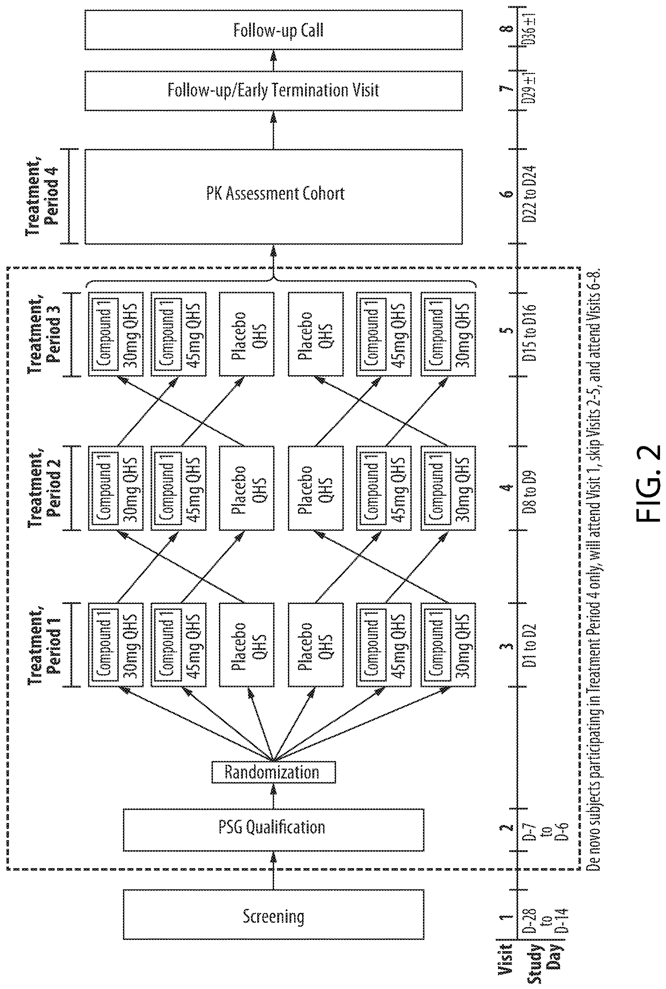

This is a double-blind, placebo-controlled, 3-way crossover study (Treatment Periods 1, 2, and 3), followed by an open-label administration of Compound 1 for pharmacokinetic purposes (Treatment Period 4). Subjects are expected to participate in Treatment Periods 1, 2 and 3. These subjects may continue into Treatment Period 4. A schematic of the study design is shown in FIG. 1. If necessary, de novo subjects may also be included in Treatment Period 4 (see FIG. 2).

### Screening (Visit 1) and Polysomnography Qualification (Visit 2)

Screening procedures will be conducted between Study Days -28 and -14, inclusive. Screening assessments may be conducted on multiple days if necessary. Subjects who pass initial screening will be asked to complete a written sleep diary for a minimum of 6 consecutive nights, and submit it (drop-off, scan and e-mail or fax it) to the clinical research coordinator (CRC) for review by Study Day -8. The CRC will review the diary and determine if the subject is eligible to continue. Continued eligibility for the study will be based on subjects having a sleep diary with the following: a minimum of 6 consecutive nights completed; typical time in bed of 7 to 9 hours; and consistent bedtime and rise time (cannot vary by more than one hour, even on weekends or days off). Subjects who do not meet the sleep diary eligibility criteria as determined by the CRC will not qualify for continued participation in the study.

Subjects who meet the sleep diary continuation criteria will return for Visit 2 (Day -7) for a one-night PSG qualification visit. The PSG qualification visit (Visit 2) will begin on Study Day -7 and will continue to Study Day -6. The CRC will determine the median habitual bedtime from their sleep diary. Subjects should arrive at the clinic about 7 hours prior to their habitual bedtime for the one-night PSG qualification visit. Subjects will receive a standard meal and be prepared for overnight PSG recording. Lights out and PSG recording will begin five hours (± 30 minutes) earlier than their mean habitual bedtime. Subjects will be required to remain in bed for eight hours, after which time the PSG recording will end, lights will turn on, and subjects will be awakened if asleep. Subjects will complete a Post Sleep Questionnaire about their subjective sleep thirty minutes after the end of the recording. Subjects will be discharged after all morning assessments have been completed at the discretion of the investigator. Subjects will have the option to remain in the clinic (e.g., to sleep at the clinic) until 9 AM.

Qualified subjects will be those who meet all screening and PSG Qualification criteria (WASO >45 minutes; Apnea-Hypopnea Index (AHI) <10; and Periodic Limb Movement Arousal Index (PLMAI) <10). They will be instructed to resume their normal sleep pattern, continue the sleep diary, and return for Treatment Period 1 (Visit 3).

### Treatment Periods 1,2, and 3 (Visits 3, 4, and 5)

Treatment Period 1 (Visit 3) begins on Study Day 1 and continues till Study Day 2. Subjects should arrive at the clinic approximately 7 hours prior to their habitual bedtime. Eligibility criteria will be confirmed and subjects will be randomized (1:1:1:1:1:1) to one of six possible treatment sequences (see FIG. 1). Predose assessments will be conducted. Subjects will receive a standard meal and be prepared for overnight PSG recording. Thirty minutes (±15 minutes) prior to lights out, blinded study drug (30 mg Compound 1, 45 mg Compound 1, or placebo) will be administered with food. Lights out and the PSG recording will begin five hours (± 30 minutes) prior to their habitual bedtime. Subjects will be required to remain in bed for eight hours, after which time the lights will turn on, and subjects will be awakened if asleep. The PSG recording will end after 5 minutes (±1 minute) of quiet wakefulness is recorded. Subjects should complete a Post Sleep Questionnaire 30 minutes after the end of the recording. Post-PSG safety assessments will be conducted per the Schedule of Assessments. Discharge from the clinic will occur at or after 6:00 AM, when the safety assessments do not reveal evidence of impairment, and at the investigator's discretion. Subjects will have the option to remain in the clinic (e.g., to sleep at the clinic) until 9:00 AM.

Treatment Period 2 (Visit 4, Study Days 8 to 9) and Treatment Period 3 (Visit 5, Study Days 15 to 16) will follow similar procedures. Subjects will resume normal sleep patterns and maintain a sleep diary during the washout periods between treatments.

With preapproval by the medical monitor, if subjects are unable to return for a visit within seven days they can be rescheduled for the following week.

Subjects who complete Treatment Period 3 and opt not to participate in Treatment Period 4, will skip Visit 6 and return for Visits 7 and 8.

### Treatment Period 4 (Visit 6): Pharmacokinetic Assessment

Approximately 10 subjects will return to the study center for Visit 6. Visit 6, Treatment Period 4, begins on the evening of Study Day 22 and continues till Study Day 24. If fewer than 10 subjects from Treatment Period 3 return for Treatment Period 4, additional de novo subjects may be enrolled. De novo subjects will undergo a limited set of screening criteria at Visit 1, skip Visits 2-5, and attend Visits 6-8 (see FIG. 2).

Subjects should arrive at approximately 7:00 PM and will be confined for two nights. Predose assessments will be conducted. A standard meal will be provided to subjects. Study drug (Compound 1, 30 mg) will be administered with food. Blood samples will be drawn at predefined time points up to and including 36 hours post-dosing on Study Day 24. Safety assessments will be obtained prior to discharge from the unit. Discharge will be at the discretion of the investigator, generally on Study Day 24.

### Follow-up Visits (Visits 7 and 8)

A follow-up visit (Visit 7) will be conducted 7 days (±1 day) after the final administration of study drug. A follow-up telephone call (Visit 8) will occur 7 days (±1 day) after the follow-up visit.

**Number of Subjects (planned):** Approximately 42 subjects will be randomized. It is expected that of those 42 subjects, a subset will continue from Treatment Period 3 to Treatment Period 4. If fewer than 10 subjects continue from Treatment Period 3 to Treatment Period 4, de novo subjects may be enrolled to ensure at least 10 subjects participate in Treatment Period 4.

### Eligibility Criteria:

### Inclusion - ALL SUBJECTS

1. Subject has provided signed informed consent before any study-specific procedures are performed.
2. Subject is willing and able to participate in the study, including all planned inpatient stays and all follow-up visits.
3. Subject is a healthy, ambulatory, man or woman aged ≥18 to ≤64 years at the Screening Visit.
4. Subject has a body weight ≥50 kg and body mass index (BMI) ≥18 and ≤ 32 kg/m2 at the Screening Visit.
5. Subject agrees to comply with the required behavioral study restrictions as outlined.
6. Female subject agrees to use one of the following methods of contraception during participation in the study and for 30 days following the last dose of study drug, unless they are postmenopausal (defined as no menses for 12 months without an alternative medical cause) and/or surgically sterile:
   - Combined (estrogen and progestogen containing) oral, intravaginal, or transdermal hormonal contraception associated with inhibition of ovulation.
   - Oral, injectable, or implantable progestogen-only hormonal contraception associated with inhibition of ovulation.
   - Intrauterine device.
   - Intrauterine hormone-releasing system.
   - Bilateral tubal occlusion.
   - Vasectomized partner.
   - Sexual abstinence (no sexual intercourse).

*Inclusion -All subjects EXCEPT de novo subjects participating in Treatment Period 4*
7. Subject has completed the Sleep Diary for a minimum of 6 consecutive days between Screening and the PSG Qualification Visit.
8. Subject has a habitual bedtime between the hours of 8:00 PM and midnight, and rise time that is consistently within a 1-hour time frame, as evidenced by Sleep Diary entries.
9. Subject spends 7 to 9 hours in bed routinely, as evidenced by Sleep Diary entries.
10. Subject meets PSG Qualification criteria following the PSG Qualification Visit including WASO >45 minutes.

*Exclusion - ALL SUBJECTS*
1. Subject has clinically significant abnormal values for hematology, clinical chemistry or urinalysis at the Screening or PSG Qualification Visit (if applicable).
2. Subject has a history of suicidal behavior, is currently at risk of suicide in the opinion of the Investigator, or has answered "YES" to questions 1, 2, 3, 4 or 5 on the C-SSRS at the Screening Visit.
3. Subject has a clinically significant abnormal physical examination finding at the Screening Visit.
4. Subject has a clinically significant abnormal 12-lead electrocardiogram (ECG) at the Screening or PSG Qualification Visit (if applicable). Note that QTcF interval of ≥450 msec in male subjects or ≥470 msec in female subjects, will be the basis for exclusion from the study. ECG may be repeated once for confirmatory purposes if initial values obtained exceed the limits specified.
5. Subject has a clinically significant history and/or presence of hepatic, renal, cardiovascular, pulmonary, gastrointestinal, hematological, immunologic, ophthalmologic, metabolic or oncological disease.
6. Subject has a history or presence of psychiatric, neurologic, or sleep/circadian disease or condition (including but not limited to epilepsy, closed head trauma with clinically significant sequela, partial onset seizures, eating disorders, sleep disorders, circadian rhythm disorders etc).
7. Subject has a recent history (within previous six months prior to screening) of substance use disorders (as judged by the Investigator)
8. Subject has a positive drug and/or alcohol test at the Screening or PSG Qualification Visit (if applicable).
9. Subject has regularly used tobacco or tobacco-containing products (cigarettes, pipes, etc.) within 30 days prior to the Screening or PSG Qualification Visit (if applicable).
10. Subject has a positive urine cotinine screen (>300 ng/mL) at the Screening or PSG Qualification Visit (if applicable).
11. Subject has a history of or current positive serological results for hepatitis B surface antigen, hepatitis C antibodies, or HIV antibodies 1 or 2.
12. Subject has participated in a clinical trial with an investigational drug or device within 30 days or 5 half-lives (if known), whichever is longer, prior to the Screening Visit.
13. Subject consumes excessive amounts of caffeine (defined as >500 mg/day) of coffee, tea, cola, and/or other caffeinated beverages within 30 days prior to the Screening Visit.
14. Subject has used known strong inhibitors and/or inducers of CYP3A4 and within the 14 days or five half-lives (whichever is longer) or consumed grapefruit juice, grapefruit, Seville oranges, or St. John's Wort or products containing these within 30 days prior to the Screening Visit.
15. Subject has had previous exposure to Compound 1 or who is known to be allergic to Compound 1 or any of its excipients, including gelatin, croscarmellose sodium, mannitol, silicified microcrystalline cellulose, and sodium stearyl fumarate.
16. Subject is an investigative site personnel or a member of their immediate family (spouse, parent, child or sibling whether biological or legally adopted).
17. Subject has a history of noncompliance, missed visits, or in the Investigator's opinion may not be a suitable candidate for participation in the study.
   *Exclusion -All subjects EXCEPT de novo subjects participating in Treatment Period 4*
18. Subject has an AHI ≥10 and/or a PLMAI ≥10 as determined by the PSG Qualification Visit.
19. Subject has worked a night shift or has flown >1 time zone within 30 days prior to the Screening Visit.
20. Subject has a Pittsburgh Sleep Quality Index (PSQI) score >5 or Epworth Sleepiness Scale score >10 at the Screening Visit.

### Planned Duration of Participation:

The estimated duration of participation for each subject is approximately 2 months which includes approximately 1 month for screening and qualification assessments, and about 1 month for the three or four treatment/washout periods and final safety follow-up. This 2-month period includes four or six overnight inpatient confinements.

For de novo subjects, the estimated duration of participation is approximately 6 weeks which includes 4 weeks for screening assessments, and about 2 weeks for Treatment Period 4 and final safety follow-up. This period includes one overnight inpatient confinement.

### Study Drug, Dosage, and Mode of Administration:

Compound 1 Capsules are available as hard gelatin capsules containing a white to off-white powder. In addition to Compound 1 Drug Substance, the Compound 1 Capsules contain croscarmellose sodium, mannitol, silicified microcrystalline cellulose, and sodium stearyl fumarate as excipients. Capsules will be available in 5-mg, 10-mg and 20-mg dose strengths. Subjects will be administered three capsules per dose (to achieve 30 mg or 45 mg, daily) at bedtime. Capsules will be taken with food and water.

Study drug administration will be under the direct supervision of the study staff or other healthcare professional.

### Reference Therapy, Dosage and Mode of Administration:

Placebo capsules are visually matched to the active capsules and are available as hard gelatin capsules containing croscarmellose sodium, mannitol, silicified microcrystalline cellulose, and sodium stearyl fumarate as excipients. Subjects will be administered three capsules per dose (for blinding purposes). Capsules will be taken with food.

Study drug administration will be under the direct supervision of the study staff or other healthcare professional.

### Duration of Treatment:

For Treatment Periods 1, 2, and 3, each subject will receive a single dose of study drug (30 mg Compound 1, 45 mg Compound 1, or placebo) on 3 separate visits; each subject will receive each dose level or placebo once. Study subjects who participate in Treatment Period 4 will receive one additional single dose of Compound 1 (30 mg) during one separate study visit.

### Criteria for Evaluation:

### Efficacy:

The efficacy of Compound 1 Capsules will be assessed by sleep efficiency and other PSG and subjective sleep data.

### Safety:

The safety and tolerability of Compound 1 Capsules will be evaluated by frequency, type, and severity of adverse events; mean changes from baseline in clinical laboratory measures, vital signs, ECGs, suicidal ideation using the C-SSRS, sleepiness using the KSS, and psychomotor performance using the DSST.

### Pharmacokinetics:

Plasma will be collected to assay for concentrations of Compound 1. The following PK parameters will be derived from the plasma concentrations (where evaluable): area under the concentration-time curve (AUC), Cmax, and tmax.

### Exploratory:

Resting state EEG data will be collected and may be analyzed using Fourier / spectral analysis for changes in EEG power following placebo or Compound 1 administration.

### Statistical Methods:

### General Considerations

Descriptive summary statistics will be provided for baseline demographics, disease characteristics, and drug exposure. Disposition including number of subjects enrolled, and percentage of subjects who discontinued from the study, along with reasons for discontinuations will be tabulated and described in listings.

Continuous data will be summarized using the following descriptive summary statistics: the number of subjects (n), mean, standard deviation (SD), median, minimum value, and maximum value.

Categorical data will be summarized using frequency counts and percentages.

Baseline value will be defined for each dosing period as the most recent non-missing measurement collected before the initial administration of study drug within the dosing period. If pre-dose measurement in a certain period is missing and no drug-free unscheduled assessments are performed before dosing in that period, the baseline of that period will be missing.

### Analysis Sets and Methods:

*The Efficacy Set* will include all subjects in the Safety Set who have post-dose PSG data. Sleep efficiency will be analyzed using a mixed effects model for repeated measures (MMRM); the model will include treatment, treatment sequence, and period as fixed effects, with adjustment for screening sleep efficiency. Model-based point estimates (ie, least squares [LS] means), 95% confidence intervals, and p-values will be reported. An unstructured covariance structure will be used to model the within-subject errors. Other continuous endpoints will be analyzed using similar methods.

*The Safety Set* will include all subjects administered study drug.

Safety endpoints included for analyses are as follow:
- Treatment-emergent adverse events (TEAEs)
- Clinical laboratory measurements (chemistry, hematology/coagulation, and urinalysis)
- ECG
- Vital signs
- C-SSRS
- KSS
- DSST

Adverse events will be classified by type, incidence, severity, and causality. The overall incidence of adverse events will be summarized by Medical Dictionary for Regulatory Activities (MedDRA) system organ class and preferred term. Data for vital signs, clinical laboratory measurements, ECG, and concomitant medication usage will also be summarized. Suicidality data collected using the C-SSRS at baseline and at each visit during the active Treatment Period will be listed for all subjects. The C-SSRS listings will include behavior type and/or category for suicidal ideation and suicidal behavior of the C-SSRS. Out-of-range safety endpoints may be categorized as low or high, where applicable. Subjects will be summarized according to treatment received.
*The PK Set* will include subjects in the Safety Set who received study drug and for whom the PK data are considered sufficient for analysis. PK parameters will be summarized using appropriate descriptive statistics and listed by subject.

### Sample Size Calculation

A sample size calculation was performed for the double-blind, placebo-controlled, crossover portion of the study (Treatment Periods 1, 2, and 3). Assuming a two-sided t-test at an alpha level of 0.05, a sample size of 31 subjects would provide 80% power to detect a difference of 8 percentage points between the Compound 1 Capsules and matching placebo groups for sleep efficiency, assuming SD of 15.3 percentage points which was estimated based on Walsh (Walsh, 2007). Assuming a non-evaluability rate of 20%, approximately 42 subjects will be randomized. A sample size calculation was not performed for the open-label Treatment Period 4. Ten subjects are considered sufficient to adequately characterize the PK and safety profile of evening dosing of Compound 1 Capsules.

The results of the experiment are provided in Example 2.

### Example 2 : Sleep Efficiency

The results from the study outlined in Example 1 (e.g., see Table 1 and/or Table 2 below) demonstrate that Compound 1 (administered as a single dose capsule of 30 mg or 45 mg in the evening, e.g. at bedtime (e.g. immediately before bedtime) was effective in increasing sleep efficiency, and was thus effective in the treatment of sleep disorders, for example, insomnia. In the study in Example 1, no serious or severe adverse events were observed. Adverse events were mild in intensity.

Sleep efficiency (SE), defined as the percentage of time in bed spent asleep as determined by PSG (Polysomnogram), increased 17.761% from baseline (median) when patients were dosed at 30 mg, and increased 20.000% from baseline (median) when patients were dosed with 45 mg. The median at baseline was 66.042%. Patients dosed with the placebo showed a 3.438% increase above baseline. Thus, patients dosed with Compound 1 showed a significant improvement in SE compared to patients given the placebo.

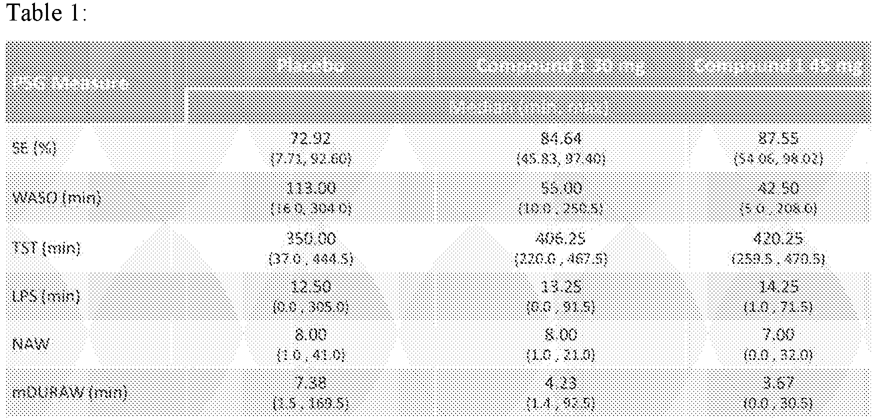

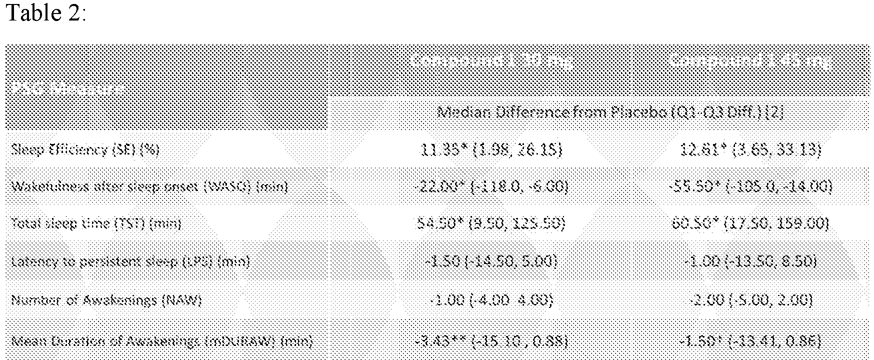

### Example 3 : Wakefulness after sleep onset

The results from the study outlined in Example 1 demonstrate that Compound 1 (administered as a single dose capsule of 30 mg or 45 mg in the evening) was effective in decreasing wakefulness after sleep onset, and was thus effective in the treatment of sleep disorders, for example, insomnia.

Wakefulness after sleep onset (WASO)(measured in minutes), defined as total wake time in minutes from persistent sleep onset to lights-on (e.g., the amount of time test subjects spent awake after initially falling asleep), decreased in patients dosed with Compound 1. Patients dosed with 30 mg of Compound 1 showed a median change from baseline of -74.75, or a decrease of 74.75 minutes (median at baseline was 134.00 minutes). Patients dosed with 45 mg of Compound 1 showed a median change from baseline of -75.25. Patients given the placebo showed only a 26.50 decrease from baseline. Thus, patients dosed with Compound 1 showed a significant decrease in WASO compared to patients given the placebo.

### Example 4 : Total sleep time

The results from the study outlined in Example 1 demonstrate that Compound 1 (administered as a single dose capsule of 30 mg or 45 mg in the evening) was effective in increasing total sleep time, and was thus effective in the treatment of sleep disorders, for example, insomnia.

Total sleep time (TST), defined as the duration of total sleep time (NREM + REM), measured in minutes, from lights-off to lights-on, increased in patients dosed with Compound 1. Patients dosed with 30 mg of Compound 1 showed a median change in TST of 85.25 minutes from baseline (median of 317.00 minutes), and patients dosed with 45 mg of Compound 1 showed a TST increase of 96.00 minutes. Patients dosed with the placebo showed only a 16.50 minute increase in TST. Thus, Compound 1 is effective in increasing total sleep time.

### Example 5 : Duration of awakenings

The results from the study outlined in Example 1 demonstrate that Compound 1 (administered as a single dose capsule of 30 mg or 45 mg in the evening) was effective in decreasing the median duration of awakenings, and was thus effective in the treatment of sleep disorders, for example, insomnia.

Mean duration of awakenings is an arithmetic mean calculated as the sum of awakenings in minutes divided by the number of awakenings. Patients dosed with 30 mg of Compound 1 were shown to have a 2.629 decrease in median duration of awakenings from baseline (where baseline was 9.364) and patients dosed with 45 mg were shown to have a 4.261 decrease in median duration of awakenings from baseline. Patients given the placebo were shown to have a 2.464 decrease from baseline.

### Example 6 : Latency to persistent sleep

The results from the study outlined in Example 1 demonstrate that Compound 1 (administered as a single dose capsule of 30 mg or 45 mg in the evening) did not impact latency to persistent sleep (LPS).

Latency to persistent sleep (LPS) is defined as the duration in minutes from lights-off to the first epoch of 20 consecutive non-wake epochs. Patients dosed with 30 mg of Compound 1 showed a 13.25 minute decrease from median baseline (28.50) and patients dosed with 40 mg of Compound 1 showed a 14.50 minute decrease from baseline. Patients dosed with the placebo showed a 12.5 minute decrease.

### Example 7: non-REM sleep and REM sleep

The results from the study outlined in Example 1 demonstrate that Compound 1 (administered as a single dose capsule of 30 mg or 45 mg in the evening) increased the number of minutes subjects slept in Stages N2 and N3 of the sleep cycle, but did not increase the amount REM sleep. Additionally, Compound 1 increased the amount of minutes of Total Sleep Time for the subjects. However, Compound 1 was not significantly different from placebo in the amount of minutes for subjective Sleep Latency (sSL). Compound 1 was effective in the treatment of sleep disorders, for example, insomnia.

Stage N2 of non-REM sleep is a period of light sleep before entering deeper sleep, where the heartbeat and breathing slow further, and muscles relax further. The body temperature drops and eye movements stop. Brain wave activity slows but is marked by brief bursts of electrical activity. Stage N3 of non-REM sleep, also referred to as Slow Wave Sleep (SWS), is the period of deep sleep. Heartbeat and breathing slow to their lowest levels during sleep and muscles are relaxed. An ideal insomnia drug would increase the amount of minutes in the N3, or optionally N2 stage of sleep without impact REM sleep (which can lead to undesirable effects well known in the art). As shown in Table 3 below, subjects administered Compound 1 at 30 mg and 45 mg showed a significant increase in the amount of minutes in Stage N2. Also shown in Table 3 below, subjects dosed with Compound 1 at 30 mg and 45 mg showed a significant increase in the amount of minutes in Stage N3. Importantly, the subjects did not show an increase in the amount of minutes in REM sleep. Thus, Compound 1 did not disrupt REM sleep.

As shown in Table 3, Wakefulness After Sleep Onset (WASO)(measured in minutes), defined as total wake time in minutes from persistent sleep onset to lights-on (e.g., the amount of time test subjects spent awake after initially falling asleep), decreased in patients dosed with Compound 1. Assessment of sWASO was significantly reduced from a median (min, max) of 20.0 (0, 300) minutes following administration of placebo to a median of 10.0 (0, 120) and 5.0 (0, 300) minutes for Compound 1 (30 and 45 mg, respectively). Total sleep time (TST), defined as the duration of total sleep time (NREM + REM), measured in minutes, from lights-off to lights-on, increased in patients dosed with Compound 1. sTST increased from a median (min, max) of 424.8 (60, 540) minutes following administration of placebo to a median of 450.0 (40, 555) and 465.0 (0, 510) minutes following administration of Compound 1 (30 and 45 mg, respectively). Administration of Compound 1 (30 and 45 mg) reduced Subjective Sleep Latency (sSL) to a median (min, max) of 15 (2, 240) and 10 (1, 60) minutes, respectively compared to a median of 15 (4,400) minutes for placebo. Assessment of Subjective Sleep Quality (sSQ) was measured on a scale of 1 (poor) to 10 (excellent). Subjects who were treated with Compound 1 (30 and 45 mg), showed a median (min, max) values of 8.0 (5, 10) and 9.0 (6, 10), respectively compared with a placebo value of 8.0 (1, 10).

Thus, Compound 1 increased the amount of minutes the subjects slept in stages N2 and N3, did not impact the REM stage of sleep, decreased the amount of time the subjects total wake time in minutes from persistent sleep onset to lights-on, increase the total sleep time, and did not significantly differ from placebo for Sleep Latency (did not cause subjects to fall asleep suddenly). Compound 1 is thus effective in the treatment of sleep disorders, for example, insomnia.

**Table 3:**

| PSG Measure | Placebo | Compound 1 (30 mg) | Compound 1 (45 mg) |
|---|---|---|---|
| Stage N1 (min) | 20.67 | 20.11 | 19.58 |
| Stage N2 (min) | 192.30 | 258.16 | 266.79 |
| Stage N3 (SWS) (min) | 56.12 | 68.38 | 74.71 |
| REM Sleep (min) | 50.59 | 50.15 | 43.54 |
| Subjective WASO (sWASO) (min) Median (min, max) | 20.0 (0, 300) | 10.0 (0, 120) | 5.0 (0, 300) |
| Subjective TST (sTST) (min) Median (min, max) | 424.8 (60, 540) | 450.0 (40, 555) | 465.0 (0, 510) |
| Subjective Sleep Latency (sSL) (min) Median (min, max) | 15.0 (4, 400) | 15.0 (2, 240) | 10.0 (1, 60) |
| Subjective Sleep Quality (sSQ) Median (min, max) | 8.0 (1, 10) | 8.0 (5, 10) | 9.0 (6, 10) |

### Equivalents and Scope

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, *e.g.,* in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims. Those of ordinary skill in the art will appreciate that various changes and modifications to this description may be made without departing from the spirit or scope of the present invention, as defined in the following claims.
Aspects of the present invention are set out in the following numbered clauses which contain the subject-matter of the clauses of the parent application as originally filed.
1. A method of treating insomnia in a subject, the method comprising administering to the subject a therapeutically effective amount of a compound having the Formula 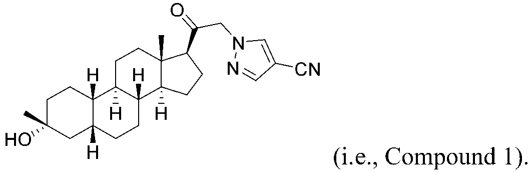
2. The method of clause 1, wherein Compound 1 is administered in the evening.
3. The method of clauses 1 or 2, wherein Compound 1 is administered prior to bedtime.
4. The method of any one of clauses 1-3, wherein Compound 1 is administered immediately before bedtime.
5. The method of any one of clauses 1-4, wherein the method increases the time of non-REM sleep.
6. The method of clauses 5, wherein the method increases the time of stage 2 sleep.
7. The method of clauses 5, wherein the method increases the time of stage 3 sleep.
8. The method of any one of clauses 1-7, wherein the method does not significantly impact the time of REM sleep.
9. The method of any one of clauses 1-8, wherein the method increases sleep efficiency.
10. The method of any one of clauses 1-9, wherein the method decreases wakefulness after sleep onset.
11. The method of any one of clauses 1-10, wherein the method increases total sleep time.
12. The method of any one of clauses 1-11, wherein Compound 1 is administered with food.
13. The method of any one of clauses 1-12, wherein the therapeutically effective amount is about 30 mg to about 45 mg.
14. The method of any one of clauses 1-13, wherein the method does not significantly impact sleep latency.
15. The method of any one of clauses 1-14, wherein Compound 1 is administered in one or more capsules.
16. The method of any one of clauses 1-15, wherein the therapeutically effective amount is administered across three capsules.
17. The method of any one of clauses 1-16, wherein the subject is treated for insomnia regardless of having an underlying condition.
18. The method of any one of clauses 1-17, wherein the subject does not have an underlying condition.
19. The method of any one of clauses 1-18, wherein the subject has an underlying condition.
20. The method of any one of clauses 1-19, wherein the insomnia is a side effect of another therapy.
21. The method of any one of clauses 1-19, wherein the insomnia is a side effect of the subject's behavior.
22. The method of any one of clauses 1-19, wherein the subject has a condition comorbid with insomnia.
23. A method of increasing slow wave sleep in a subject comprising administering to a subject with a neurodegenerative disease or a central nervous system disorder a pharmaceutical composition comprising an effective amount of Compound 1 or a derivative thereof and a pharmaceutically acceptable carrier or excipient.
24. A method of increasing slow wave sleep in a subject comprising administering to a subject a pharmaceutical composition comprising an effective amount of Compound 1 or a derivative thereof and a pharmaceutically acceptable carrier or excipient.
25. A method of increasing slow wave sleep in a subject without significantly impacting REM sleep comprising administering to a subject a pharmaceutical composition comprising an effective amount of Compound 1 or a derivative thereof and a pharmaceutically acceptable carrier or excipient.
26. The method of any one of clauses 1-25, wherein the effective amount of Compound 1 is administered once every 24-48 hours.

## Claims

1. Compound 1: 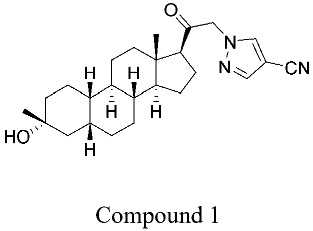 or a pharmaceutically acceptable salt thereof,
for use in a method of treating insomnia in a subject,
wherein the subject has a condition comorbid with insomnia.

2. A pharmaceutical composition for use in a method of treating insomnia in a subject, wherein the pharmaceutical composition comprises Compound 1: 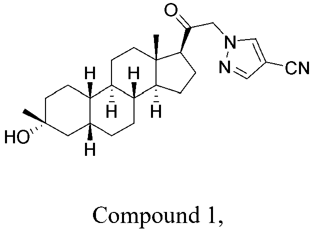 and a pharmaceutically acceptable excipient, and
wherein the subject has a condition comorbid with insomnia.

3. Compound 1, or a pharmaceutically acceptable salt thereof, for use according to claim 1 or the pharmaceutical composition for use according to claim 2, wherein the condition comorbid with insomnia is a CNS-related disorder.

4. Compound 1, or a pharmaceutically acceptable salt thereof, or the pharmaceutical composition for use according to claim 3, wherein the CNS-related disorder is: tremor, for example, essential tremor; depression, for example, postpartum depression; or an anxiety disorder.

5. Compound 1, or a pharmaceutically acceptable salt thereof, or the pharmaceutical composition for use according to claim 4, wherein the CNS-related disorder is postpartum depression.

6. Compound 1, or a pharmaceutically acceptable salt thereof, for use according to any one of claims 1 or 3 to 5, or the pharmaceutical composition for use according to any one of claims 2 to 5, wherein Compound 1 is administered once every 24-48 hours.

7. Compound 1, or a pharmaceutically acceptable salt thereof, or the pharmaceutical composition for use according to claim 6, wherein Compound 1 is administered once every 24 hours.

8. Compound 1, or a pharmaceutically acceptable salt thereof, for use according to any one of claims 1 or 3 to 7, or the pharmaceutical composition for use according to any one of claims 2 to 7, wherein Compound 1 is administered:
(i) at about 10 mg to about 100 mg;
(ii) at about 20 mg to about 50 mg; or
(iii) at about 30 mg to about 45 mg.

9. Compound 1: 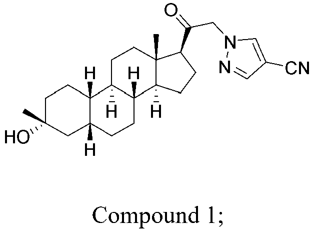 or a pharmaceutically acceptable salt thereof,
for use in a method for treating insomnia comorbid with postpartum depression (PPD) in a subject,
wherein Compound 1 is administered once every 24 hours and at about 20 mg to about 50 mg.

10. A pharmaceutical composition for use in a method for treating insomnia comorbid with postpartum depression (PPD) in a subject, wherein the pharmaceutical composition comprises Compound 1: 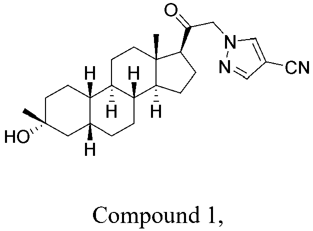 and a pharmaceutically acceptable excipient;
wherein the pharmaceutical composition is administered once every 24 hours; and
wherein the pharmaceutical composition comprises about 20 mg to about 50 mg of Compound 1.

11. Compound 1, or a pharmaceutically acceptable salt thereof, for use according to any one of claims 1 or 3 to 9, or the pharmaceutical composition for use according to any one of claims 2 to 8 or 10, wherein the method:
(a) increases the time of non-REM sleep;
(b) increases the time of stage 2 sleep;
(c) increases the time of stage 3 sleep;
(d) increases sleep efficiency;
(e) decreases wakefulness after sleep onset;
(f) increases total sleep time; and/or
(g) does not significantly impact sleep latency.

12. Compound 1, or a pharmaceutically acceptable salt thereof, for use according to any one of claims 1, 3 to 9 or 11, wherein Compound 1 or the pharmaceutically acceptable salt of Compound 1 is administered in one or more capsules.

13. Compound 1, or a pharmaceutically acceptable salt thereof, for use according to any one of claims 1, 3 to 9, 11 or 12, wherein Compound 1 or the pharmaceutically acceptable salt of Compound 1 is administered with food.

14. The pharmaceutical composition for use according to any one of claims 2 to 8, 10 or 11, wherein the pharmaceutical composition is administered in one or more capsules.

15. The pharmaceutical composition for use according to any one of claims 2 to 8, 10, 11 or 14, wherein the pharmaceutical composition is administered with food.
